# EUROPÄISCHE PATENTSCHRIFT

(11) **EP 0 906 970 B1**
(45) Veröffentlichungstag und Bekanntmachung des Hinweises auf die Patenterteilung: **14.03.2001**
(21) Anmeldenummer: 98117801.5
(22) Anmeldetag: 19.09.1998
(51) Int. Cl.: C25B 3/00, C07C 303/32, C07C 309/04

(54) **Verfahren zur Herstellung von Silberverbindungen**
Process for the preparation of silver compounds
Procédé de préparation de composés d'argent

(30) Priorität: 02.10.1997 DE 19743613
(43) Veröffentlichungstag der Anmeldung: 07.04.1999
(73) Patentinhaber: Goldschmidt AG, 45127 Essen (DE)
(72) Erfinder: Guhl, Dieter Dr., 67346 Speyer (DE); Honselmann, Frank, 67366 Weingarten (DE)

(56) Entgegenhaltungen:
- WO-A-85/03530
- US-A- 5 491 247
- PATENT ABSTRACTS OF JAPAN vol. 017, no. 659 (C-1137), 7. Dezember 1993 & JP 05 213854 A (MITSUI PETROCHEMICAL INDUSTRY), 24. August 1993

## Beschreibung

Gegenstand der Erfindung ist ein membranfreies Verfahren zur Herstellung von Silberverbindungen.

Elektrolyte zur chemischen Versilberung mit Silbernitrat und Thioharnstoff oder ähnlichen Verbindungen werden als Elektrolyte zu Metallabscheidung eingesetzt. Speziell bei Silberelektrolyten sind jedoch Alternativen zu Silbernitrat oder Silbercyanid erwünscht.

Elektrolyte zur chemischen Versilberung mit Silbernitrat und Thioharnstoff oder ähnlichen Verbindungen neigen beim Eintrocknen zu explosionsartigen Zersetzungen.

Zur galvanischen Abscheidung verwendet man praktisch nur alkalische cyanidhaltige Elektrolyte mit Silbercyanid. Diese Elektrolyte haben den Nachteil der hohen Giftigkeit. Darüber hinaus werden Abdecklacke von elektronischen Bauteilen durch diese Elektrolyte angegriffen. Weiterhin lassen diese Elektrolyte nur relativ geringe Stromdichten/Abscheidungsgeschwindigkeiten zu. Salpetersaure Elektrolyte mit Silbernitrat sind zur galvanischen Metallabscheidung nicht geeignet, da das Nitrat kathodisch reduktiv zerstört wird.

Andere, im technischen Maßstab erhältliche Silbersalze, wie Silberchlorid oder Silbersulfat sind zur Herstellung von Chlorid- oder Sulfatelektrolyten nicht geeignet, da die Wasserlöslichkeit dieser Silberverbindungen zu gering ist.

Demgegenüber ist die gute Wasserlöslichkeit von Silbermethansulfonat bekannt, so daß diese Verbindung und spezielle Silber-Alkyl- oder Arylsulfonate als Elektrolyt zur galvanischen Abscheidung von Silber eine interessante Alternative zu dem bekannten Elektrolyten darstellen.

WO 85/03530 beschreibt ein Verfahren zur Herstellung von Metallcarboxylaten von Schwermetallen. Auf S. 6, Z. 14 ist auch Silber als eines der Schwermetalle genannt. Auf S. 14, Z. 27 bis S. 15, Z. 5 ist weiterhin beschrieben, daß auch Sulfonsäuresalze, z. B. von p-Toluolsulfonsäure mit diesem Verfahren hergestellt werden können. Keines der Ausführungsbeispiele zeigt jedoch die Anwendung von Silber.

In dem die S. 3 und 4 übergreifenden Abs. wird festgehalten, daß der Elektrolyt eine ionische Komponente enthalten soll, die leicht in Anionen und Kationen dissoziiert, wobei insbesondere Alkalimetallhalogenide, insbesondere Natriumchlorid vorgeschlagen werden.

Bekanntermaßen sind Silberhalogenide in wäßrigen Lösungen schwer löslich, so daß im Falle der anodischen Auflösung von Silber in einem Natriumchlorid-haltigen Elektrolyten unmittelbar Silberchlorid ausfallen würde.

Im US-Patent 5,618,404 wird ein elektrolytisches Verfahren zur Herstellung von Blei- und Zinnsulfonaten beschrieben, das jedoch eine aufwendige Trennung des Anodenraums von dem Kathodenraum durch Kationen- und Anionenaustauschermembranen beinhaltet.

Aus JP 0 521 38 54 A2 (CA 119:270800) ist ein Verfahren zur Herstellung von Silbersulfonatsalzen bekannt. Eine Lösung von Silbercarbonat in Methylcyanid wird mit einer Lösung von Methansulfonsäure in Methylcyanid umgesetzt, um 93 %iges Silbermethylsulfonat zu ergeben. Weiterhin ist auch bekannt, Silbermethansulfonat aus wäßriger Methansulfonsäure und Silbercarbonat bzw. Silberoxid oder aus wäßriger Methansulfonsäure, Silberpulver und gegebenenfalls Wasserstoffperoxid herzustellen (US 5,491,247 A). Die vorgenannten Verfahren sind jedoch sehr kostenaufwendig, da man in der Regel zunächst das Silber in Salpetersäure löst, um das Silbercarbonat bzw. Silberoxid herzustellen und diese Verbindung dann mit Methansulfonsäure umzusetzen. Die Herstellung aus dem Metall mit Wasserstoffperoxid ist zwar kostengünstiger, jedoch ist für akzeptable Reaktionszeiten eine große Metalloberfläche erforderlich. Dies bedingt die Notwendigkeit des Einsatzes von teuerem Metallpulver. Darüber hinaus besteht ein gravierender Nachteil darin, daß mehr oder weniger große Mengen von Methansulfonsäure zu Schwefelsäure und Kohlendioxid zersetzt werden, wodurch relativ teures Wasserstoffperoxid verbraucht wird.

Die Aufgabe der vorliegenden Erfindung gegenüber dem obengenannten Stand der Technik besteht somit in einem verbesserten Verfahren zur Herstellung von Silberverbindungen, insbesondere Silbersulfonaten.

Die vorgenannte Aufgabe wird gelöst durch ein Verfahren zur Herstellung von Silberverbindungen der allgemeinen Formel (I)

RSO₃Ag (I)

wobei
R für einen gegebenenfalls substituierten, geradkettigen oder verzweigten, gesättigten, einfach oder mehrfach ungesättigten Alkyl- oder Alkenylrest mit 1 bis 9 C-Atomen oder einen gegebenenfalls substituierten Arylrest mit 6 bis 12 C-Atomen steht,
dadurch gekennzeichnet, daß man Säuren der allgemeinen Formel (II)

RSO₃H (II)

wobei
R die obengenannte Bedeutung hat,
in einer membranfreien Elektrolysezelle mit metallischem Silber als Anode einer elektrolytischen Auflösung der Anode unterwirft.

Mit Hilfe der vorliegenden Erfindung ist es möglich, Silberverbindungen der allgemeinen Formel (I) elektrochemisch herzustellen, wobei die Herstellungskosten günstiger sind, als bei den im Stand der Technik beschriebenen chemischen Verfahren. Die technische Realisierung von Membranelektrolysezellen ist sehr aufwendig; die verwendeten Membranen in der Regel sehr teuer. Kurze Standzeiten verkürzen oft den Prozeß zusätzlich.

Die mit Hilfe der vorliegenden Erfindung erhältlichen Lösungen der Silberverbindungen der allgemeinen Formel (I) können direkt als Elektrolyt für die galvanische Abscheidung eingesetzt werden und bedürfen daher nicht notwendigerweise der Aufarbeitung zu den festen Verbindungen. Auch die Lösungen dieser Silberverbindungen sind über lange Zeit stabil.

Wenn im Sinne der vorliegenden Erfindung substituierte Alkyl-, Alkenyl- oder Arylsulfonsäuren eingesetzt werden, so sind hier vorzugsweise halogen-, hydroxy-, amino-, sulfo- oder carboxylsubstituierte Derivate hervorzuheben. Im Sinne der vorliegenden Erfindung werden somit insbesondere Hydroxysulfonsäure oder Sulfobernsteinsäure mit eingeschlossen. Da die Verwendung von Silbermethansulfonsäure und/oder Silbertoluolsulfonsäure als Elektrolyt zur chemischen Versilberung bereits im Stand der Technik bekannt ist, wird das erfindungsgemäße Verfahren vorzugsweise zur Herstellung dieser Verbindungen, bzw. deren Lösungen eingesetzt. Dementsprechend ist es erforderlich, als Säure der allgemeinen Formel (II) direkt Methansulfonsäure oder Toluolsulfonsäure einzusetzen.

Besonders bevorzugt im Sinne der vorliegenden Erfindung werden wäßrige Lösungen der vorgenannten Säuren der allgemeinen Formel (II), insbesondere in einer Konzentration von 5 bis 70 Gew.-% oder beispielsweise in einer Konzentration von 30 %iger Sulfonsäure eingesetzt. Bei der Durchführung der elektrolytischen Zersetzung des metallischen Silbers kann daher der Elektrolyt von Zeit zu Zeit im Anodenbereich entnommen werden und mit frischer wäßriger Sulfonsäure, beispielsweise Methansulfonsäure ergänzt werden.

Bei der Auswahl der Materialien, die für die metallische Silberanode eingesetzt werden, spielt der Preis des Materials eine wesentliche Rolle. Dementsprechend ist es im Sinne der vorliegenden Erfindung besonders bevorzugt, metallisches Silber in Form von Pulver, Granalien, Nadeln, Schrot, Draht, Stäben, Wolle, Folien oder Blech einzusetzen. Besonders hervorzuheben sind an dieser Stelle die kostengünstigen Granalien, die gegenüber Pulver einen besonderen Preisvorteil aufweisen.

Die membranfreie Elektrolysezelle kann nach üblichen Anleitungen hergestellt und nach bekannten Verfahren betrieben werden. Besonders bevorzugt im Sinne der vorliegenden Erfindung ist es daher, die Reaktion bei einer Temperatur im Bereich 15 °C bis 80 °C bei einer Spannung von 2 V bis 20 V und anodischer Stromdichte von 0,5 A/dm² bis 15 A/dm² kontinuierlich durchzuführen.

Um eine gleichzeitige kathodische Abscheidung des Silbers zu vermeiden, müssen Anodenraum und Kathodenraum auf konstruktive weise voneinander getrennt werden. Mit diesen Einrichtungen wird die Migration der Silberionen im elektrischen Feld stark gehemmt.

Die mit Hilfe der vorliegenden Erfindung erhältlichen Silberverbindungen der allgemeinen Formel (I) werden üblicherweise in der galvanischen Industrie in Form von wäßrigen Lösungen eingesetzt. Dementsprechend ist es nicht erforderlich, die Verbindungen in kristalliner, das heißt in fester Form herzustellen. Es bietet sich vielmehr an, die im Laufe des elektrolytischen Verfahrens auftretenden wäßrigen Lösungen direkt dem Elektrolysebad zu entnehmen, wenn die gewünschte Konzentration erreicht ist, um anschließend das Elektrolysebad mit frischer Säure der allgemeinen Formel (II) gegebenenfalls in wäßriger Lösung zu ersetzen.

### Ausführungsbeispiele:

Als membranfreie Elektrolysezelle diente ein 500 ml Becherglas. Als Anode wurden handelsübliche Silbergranalien verwendet. Die Granalienschüttung wurde über einen Platindraht anodisch geschaltet. Als Kathode diente eine Platinelektrode (geeignet sind auch andere inerte Metalle oder Silber, welches zwar langsam angegriffen wird, die Lösung aber nicht verunreinigt).

Die Anodenoberfläche betrug etwa 0,25 dm². Die Kathodenoberfläche etwa 1 cm². Als Elektrolyt wurde eine wäßrige Lösung mit etwa 30 % Methansulfonsäure in vollentsalztem Wasser eingesetzt. Bei dieser Konzentration an Säure löste sich elektrochemisch am meisten Silber.

Bei einem Strom von etwa 1 A und einer Spannung von etwa 7V wurde das Silber dann anodisch gelöst. Der ungekühlte Elektrolyt erwärmte sich hierbei auf bis zu 51 °C. Um eine gleichzeitige kathodische Abscheidung des Silbers zu vermeiden, wurden Anodenraum und Kathodenraum voneinander getrennt. Hier wurden Anode und Kathode konstruktiv räumlich voneinander getrennt.

Von Zeit zu Zeit wurde dann der mit Silber angereicherte Elektrolyt im Anodenbereich entnommen und mit der 30 %igen Methansulfonsäure ergänzt.

Die entnommenen Silbermethansulfonatlösungen enthielten bis zu 405 g/l Ag und etwa 5 bis 10 g/l CH₃SO₃H. Ein Gehalt von etwa 350 g/l Ag und kleiner 50 g/l CH₃SO₃H konnte reproduzierbar erreicht werden.

Die Stromausbeute betrug im Durchschnitt etwa 70 %.

Die Lösungen sind wasserklar und mehrere Monate stabil.

## Patentansprüche

1. Verfahren zur Herstellung von Silberverbindungen der allgemeinen Formel (I)
RSO₃Ag (I)
wobei
R für einen gegebenenfalls substituierten, geradkettigen oder verzweigten, gesättigten, einfach oder mehrfach ungesättigten Alkyl- oder Alkenylrest mit 1 bis 9 C-Atomen oder einen gegebenenfalls substituierten Arylrest mit 6 bis 12 C-Atomen steht,
dadurch gekennzeichnet, daß man Säuren der allgemeinen Formel (II)
RSO₃H (II)
wobei
R die obengenannte Bedeutung hat,
in einer membranfreien Elektrolysezelle mit metallischem Silber als Anode einer elektrolytischen Auflösung der Anode unterwirft.

2. Verfahren nach Anspruch 1, dadurch gekennzeichnet, daß man halogensubstituierte Säuren der allgemeinen Formel (II) einsetzt.

3. Verfahren nach Anspruch 1, dadurch gekennzeichnet, daß man als Säuren der allgemeinen Formel (II) Methansulfonsäure und/oder Toluolsulfonsäure einsetzt.

4. Verfahren nach Anspruch 1 bis 3, dadurch gekennzeichnet, daß man eine wäßrige Lösung der Säuren der allgemeinen Formel (II), insbesondere in einer Konzentration von 5 bis 70 Gew.-% einsetzt.

5. Verfahren nach einem der Ansprüche 1 bis 4, dadurch gekennzeichnet, daß man metallisches Silber in Form von Pulver, Granalien, Nadeln, Schrot, Draht, Stäben, Wolle, Folien oder Blechen einsetzt.

6. Verfahren nach einem der Ansprüche 1 bis 5, dadurch gekennzeichnet, daß man die Reaktion bei der Temperatur im Bereich von 15 °C bis 80 °C im Verlauf bei einer Spannung von 2 V bis 20 V und anodischer Stromdichte von 0,5 A/dm² bis 15 A/dm² kontinuierlich durchführt.

7. Verfahren nach einem der Ansprüche 1 bis 6, zur Herstellung von Lösungen von Silberverbindungen der allgemeinen Formel (I), wobei man die Lösungen dem Reaktionsansatz entnimmt und mit frischer Säure der allgemeinen Formel (II) kontinuierlich oder diskontinuierlich ersetzt.

## Claims

1. A process for preparing silver compounds of the general formula (I)
RSO₃Ag (I)
where
R is an unsubstituted or substituted, linear or branched, saturated, monounsaturated or polyunsaturated alkyl or alkenyl radical having 1 to 9 carbon atoms or an unsubstituted or substituted aryl radical having 6 to 12 carbon atoms,
which comprises subjecting acids of the general formula (II)
RSO₃H (II)
where
R has the abovementioned meaning, to an electrolytic dissolution of the anode in a membraneless electrolysis cell having metallic silver as anode.

2. The process as claimed in claim 1, wherein use is made of halogen-substituted acids of the general formula (II).

3. The process as claimed in claim 1, wherein, as acids of the general formula (II), use is made of methanesulfonic acid and/or toluenesulfonic acid.

4. The process as claimed in claims 1 to 3, wherein an aqueous solution of the acids of the general formula (II) is used, in particular at a concentration of 5 to 70% by weight.

5. The process as claimed in one of claims 1 to 4, wherein metallic silver is used in the form of powder, granules, needles, shot, wire, rods, wool, foils or sheets.

6. The process as claimed in one of claims 1 to 5, wherein the reaction is carried out continuously at a temperature in the range from 15°C to 80°C at a voltage of 2 V to 20 V and anodic current density of 0.5 A/dm² to 15 A/dm².

7. The process as claimed in one of claims 1 to 6, for preparing solutions of silver compounds of the general formula (I), where the solutions are taken off from the reaction batch and continuously or batchwise replaced with fresh acid of the general formula (II).

## Revendications

1. Procédé pour la préparation de composés d'argent de formule générale (I)
RSO₃Ag (I)
où
R représente un radical alkyle ou alcényle comprenant 1 à 9 atomes de carbone, le cas échéant substitué, linéaire ou ramifié, saturé, monoinsaturé ou polyinsaturé, ou un radical aryle comprenant 6 à 12 atomes de carbone, le cas échéant substitué,
caractérisé en ce qu'on soumet des acides de formule générale (II)
RSO₃H (II)
où
R a la signification susmentionnée,
dans une cellule d'électrolyse exempte de membrane avec de l'argent métallique comme anode, à une dissolution électrolytique de l'anode.

2. Procédé selon la revendication 1, caractérisé en ce qu'on utilise des acides de formule générale (II) substitués par halogène.

3. Procédé selon la revendication 1, caractérisé en ce qu'on utilise comme acides de formule générale (II) de l'acide méthanesulfonique et/ou de l'acide toluènesulfonique.

4. Procédé selon la revendication 1 à 3,
caractérisé en ce qu'on utilise une solution aqueuse des acides de formule générale (II), en particulier en une concentration de 5 à 70% en poids.

5. Procédé selon l'une quelconque des revendications 1 à 4, caractérisé en ce qu'on utilise de l'argent métallique sous forme de poudre, de grenailles, d'aiguilles, de mitraille, de fil, de barres, de laine, de feuillards ou de plaques.

6. Procédé selon l'une quelconque des revendications 1 à 5, caractérisé en ce qu'on effectue la réaction de manière continue à la température dans la plage de 15°C à 80°C pendant le déroulement, à une tension de 2 V à 20 V et une densité de courant anodique de 0,5 A/dm² à 15 A/dm².

7. Procédé selon l'une quelconque des revendications 1 à 6, pour la préparation de solutions de composés d'argent de formule générale (I), les solutions étant prélevées de la charge réactionnelle et remplacées par de l'acide frais de formule générale (II), de manière continue ou discontinue.
